# EUROPÄISCHE PATENTANMELDUNG

(11) **EP 0 689 800 A2**
(43) Veröffentlichungstag der Anmeldung: **03.01.1996**
(21) Anmeldenummer: 95109865.6
(22) Anmeldetag: 23.06.1995
(51) Int. Cl.: A61B 17/72, A61B 17/74

(54) **Fixierungs- und Positionierungssystem für intramedulläre Kraftträger**

(30) Priorität: 01.07.1994 DE 4423210
(71) Anmelder: Kessler, Sigurd, Dr., D-82178 Puchheim (DE)
(72) Erfinder: Kessler, Sigurd, Dr., D-82178 Puchheim (DE)
(74) Vertreter: Finsterwald, Manfred, Dipl.-Ing., Dipl.-Wirtsch.-Ing.

(57) **Zusammenfassung**

Es wird ein Fixierungs- und Positionierungssystem für intramedulläre Kraftträger beschrieben, das zumindest zwei gegenseitig beabstandete Fixiereinheiten verwendet, wobei jede Fixiereinheit als bezüglich des Knochens festlegbare, dem jeweiligen Kraftträger durchdringungsfrei halternde, Rotations-, Kippungs- und Verkürzungseffekte ausschließende Einheit ausgebildet ist.

## Beschreibung

Die Erfindung betrifft ein Fixierungs- und Positionierungssystem für intramedulläre Kraftträger, insbesondere für vorbohrungsfrei in das jeweilige Knochenrohr einbringbare Marknägel, mit zumindest zwei gegenseitig beabstandeten Fixiereinheiten.

Intramedulläre Osteosynthesen werden in der Praxis in großem Umfange verwendet, und dabei ist es bekannt und üblich, zur Fixierung des jeweiligen Kraftträgers diesen Kraftträger durchdringende und bezüglich des jeweiligen Knochens befestigbare Elemente zu verwenden.

Aufgabe der vorliegenden Erfindung ist es, die Möglichkeiten der intramedullären Osteosynthesen zu verbessern, insbesondere die technische Handhabung zu vereinfachen und die Anwendung auch bei solchen Frakturformen zu gewährleisten, bei denen derartige Systeme bisher nicht eingesetzt werden konnten. Insbesondere ist es auch Ziel der Erfindung, die Einsatzmöglichkeiten von Kraftträgern bzw. Marknägeln zu verbessern, die nur einen geringen Querschnitt aufweisen und auch vorbohrungsfrei eingebracht werden können.

Der wesentliche Lösungsansatz der Erfindung zur Verbesserung intramedullärer Osteosynthesen besteht darin, daß jede Fixiereinheit als bezüglich des Knochens festlegbare, dem jeweiligen Kraftträger durchdringungsfrei halternde, Rotations-, Kippungs- und Verkürzungseffekte ausschließende Einheit ausgebildet ist.

Durch die Erfindung, deren verschiedene Ausgestaltungen in den Unteransprüchen angegeben sind, wird es auch möglich, Kraftträger bzw. Marknägel von vergleichsweise geringem Querschnitt zu verwenden, da durch die Fixiereinheiten keinerlei Schwächung derartiger Kraftträger erfolgt und damit auch die sonst gegebene Bruchgefahr ausgeschaltet wird.

Außerdem gewährleisten die erfindungsgemäßen Maßnahmen der Klemmung des Kraftträgers dessen verdreh-, verrutsch- und kippfeste Positionierung. Ein besonders wesentlicher Vorteil besteht darin, daß die Fixierung des Kraftträgers auch in Gelenknähe erfolgen kann und somit der Einsatz dieser Technik auch bei Frakturen möglich ist, die sich bis in den Gelenkbereich erstrecken.

Weitere Vorteile der Erfindung, insbesondere auch im Zusammenhang mit den in den Unteransprüchen angegebenen Ausführungsvarianten, werden nachfolgend anhand von Ausführungsbeispielen unter Bezugnahme auf die Zeichnung erläutert; in der Zeichnung zeigt:
- Figur 1: eine schematische Darstellung einer erfindungsgemäßen Fixierung in unmittelbarer Nähe des Kraftträgerendes,
- Figur 2: eine schematische Darstellung einer mit Klemmbacken arbeitenden Fixiereinheit,
- Figur 3: eine modifizierte Klemmbackenanordnung der Fixiereinheit nach Figur 2,
- Figur 4: eine mit Klemmplatten arbeitende Fixiereinheit,
- Figur 5: eine Schnittdarstellung senkrecht zur Kraftträgerachse der Fixiereinheit nach Figur 4,
- Figur 6: eine modifizierte Fixiereinheit mit Klemmbacken,
- Figur 7: eine schematische Darstellung einer Marknagel-Endfixierung mittels einer Köchereinheit,
- Figur 8: eine schematische Darstellung einer mit einem Klemmkonus arbeitenden Fixiereinheit,
- Figur 9: eine schematische Darstellung einer Kombination zweier endseitig vorgesehener Klemmeinheiten, wobei einendig ein Schraubgewinde verwendet ist,
- Figur 10: eine schematische Darstellung einer durch eine Dreipunktbiegung realisierten Fixiereinheit,
- Figur 11: die Fixiereinheit nach Figur 10 im Zusammenwirken mit einer weiteren endseitig vorgesehenen Fixiereinheit,
- Figur 12: eine schematische Schnittdarstellung zur Erläuterung einer mit Klippung arbeitenden Fixiereinheit,
- Figur 13: eine schematische Darstellung einer Fixiereinheit mit Marknagel-Endverschraubung,
- Figur 14: eine schematische Teilschnitt-Darstellung einer Marknagel-Fixierung mittels arretierter Spreizer,
- Figur 15: eine schematische Darstellung einer Marknagel-Fixierung mittels Führungs- und Klemmrohren,
- Figur 16: eine schematische Darstellung einer Kombination von Fixiereinheiten mit einer Zuggurtungsplatte,
- Figur 17: eine schematische Darstellung einer speziellen Fixierung im Hüftknochenbereich, die
- Figuren 18 und 19: schematische Darstellungen eines Kraftträgers mit einem Führungssystem zur Auffädelung von Knochenfragmenten, und
- Figur 20: eine schematische Darstellung einer Anordnung gebündelter Kraftträger.

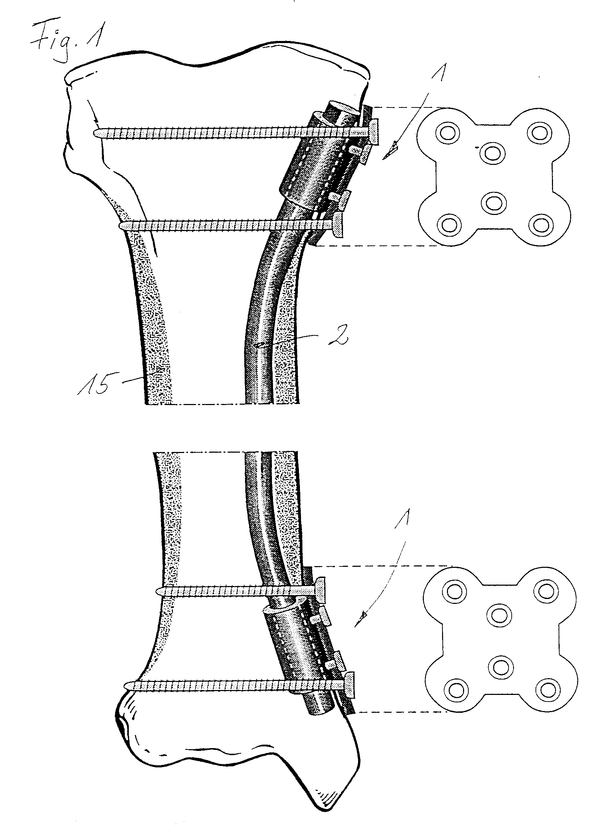

Figur 1 zeigt einen in einem Knochen 15 angeordneten Kraftträger bzw. Marknagel 2, der in unmittelbarer Nähe der jeweiligen Nagelenden mittels Fixiereinheiten 1 in der Weise gehalten ist, daß Rotations-, Kippungs- und Verkürzungseffekte ausgeschlossen sind. Die Möglichkeit der Fixierung in unmittelbarer Nähe des jeweiligen Nagelendes gestattet die Versorgung von Frakturen über die maximal denkbare Knochenstrecke.

Die in den folgenden Ausführungsbeispielen beschriebenen Fixiereinheiten eignen sich sowohl für herkömmlche Marknägel, sind aber insbesondere und vorzugsweise bestimmt für die ungebohrte, intramedulläre Schienung, bei der der Kraftträger nur einen vergleichsweise geringen Querschnitt aufweist. Dazu sind die erfindungsgemäßen Fixiereinheiten in der Weise ausgebildet, daß durch sie keine mechanische Schwächung des jeweiligen Kraftträgers erfolgt und somit stets die mechanischen Anforderungen hinsichtlich Belastbarkeit und Frakturruhigstellung erfüllt werden können.

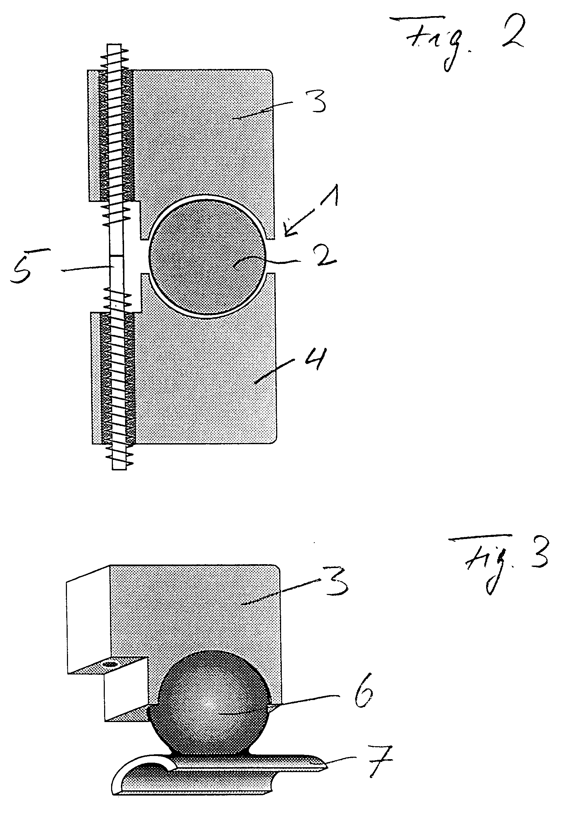

Figur 2 zeigt eine Fixiereinheit 1, die aus zwei Klemmbacken 3, 4 besteht, welche mittels einer Schraubspindel 5 mit gegenläufigem Gewinde relativ zueinander bewegt werden können. Durch Betätigung der Schraubspindel 5 kann somit der jeweilige Kraftträger 2 form- und kraftschlüssig gefaßt und damit durch entsprechende Befestigung dieser Fixiereinheit 1 am jeweiligen Knochen eindeutig positioniert und gegen Rotations-, Kippungs- und Verkürzungseffekte gesichert werden.

Die Detailansicht nach Figur 3 zeigt eine Modifikation der Anordnung nach Figur 2 dergestalt, daß zur Gewährleistung einer noch besseren kraft- und formschlüssigen Anpassung der Klemmbacken an den Kraftträger 2 zumindest einer der Klemmbacken mit einer Klemmschale 7 versehen ist, die bezüglich des Klemmbackens 3 beweglich, insbesondere über ein Kugelgelenk 6, gelagert ist.

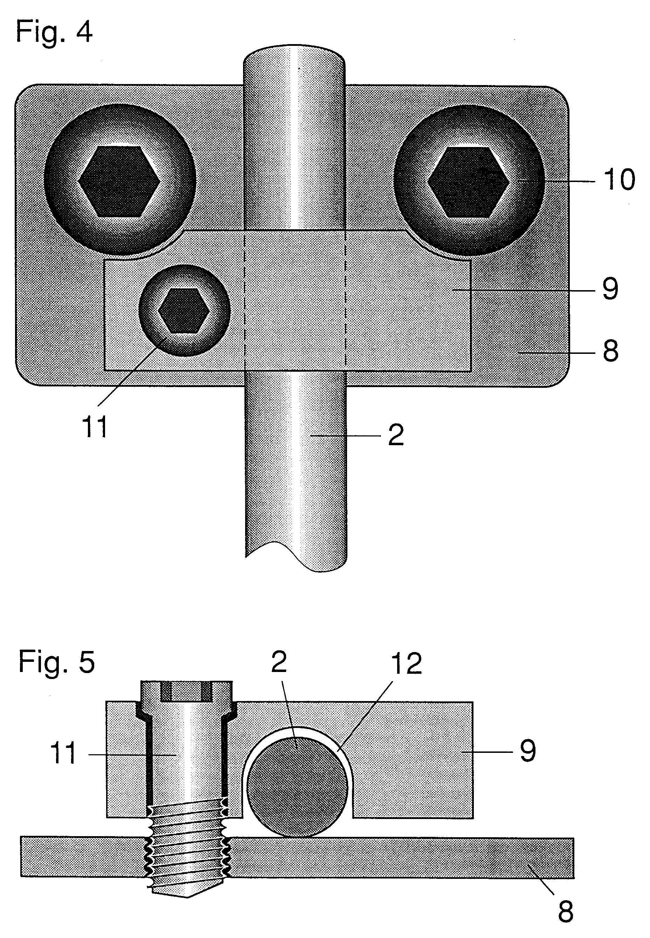

Figur 4 zeigt in Draufsicht eine Fixiereinheit, bei der der Kraftträger 2 mittels zweier Klemmplatten, nämlich einer Grundplatte 8 und einer Druckplatte 9, form- und kraftschlüssig gehalten ist. Die Grundplatte 8 wird mittels Plattenschrauben 10 am jeweiligen Knochen fixiert, und die Druckplatte 9 wird mittels zumindest einer Klemmschraube 11 gegen die Grundplatte 8 unter gleichzeitiger Festspannung des Kraftträgers 2 verspannt.

Figur 5 zeigt die gegenseitige Verspannung von Grundplatte 8 und Druckplatte 9 in der Querschnittdarstellung. Anstelle einer einzigen Klemmschraube kann auch eine Klemmschraubenbefestigung beiderseits des Kraftträgers 2 vorgesehen sein.

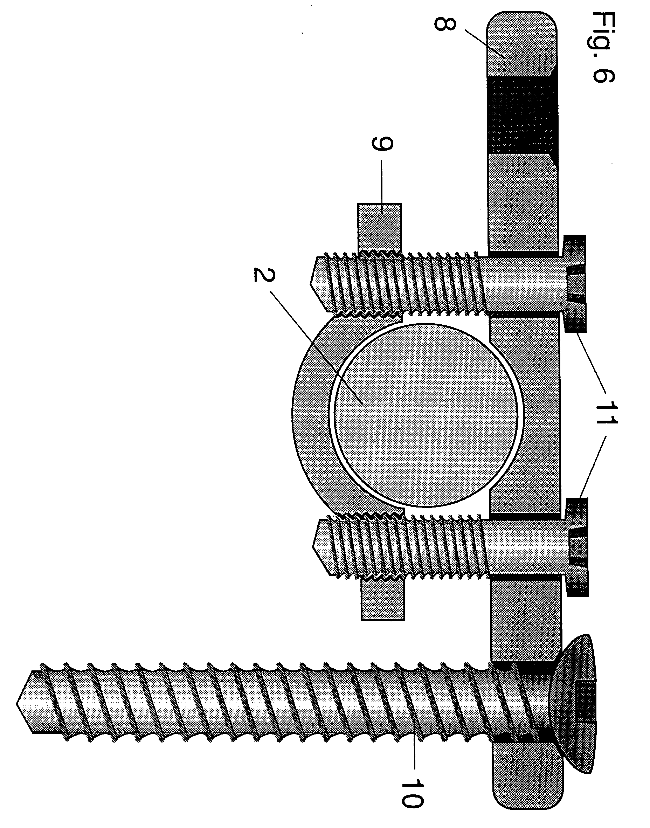

Figur 6 zeigt eine weitere Variante einer Klemmplatten- oder Schellenbefestigung des Kraftträgers 2. Auch bei dieser Ausführungsform ist die Grundplatte 8 mittels geeigneter Plattenschrauben am Knochen zu fixieren, und die form- und kraftschlüssige Halterung des Kraftträgers oder Marknagels 2 wird mittels einer als Klemmschelle ausgebildeten Druckplatte 9 realisiert, die mittels zweier Schraubenpaare unter Zwischenschluß des Kraftträgers 2 gegen die Grundplatte 8 verspannbar ist. Diese Verspannung kann dabei problemlos verdreh- und verrutschfest sowie kippfest gestaltet werden, da die Marknagelklemmung von der Befestigung der Grundplatte bezüglich des Knochens völlig unabhängig ist.

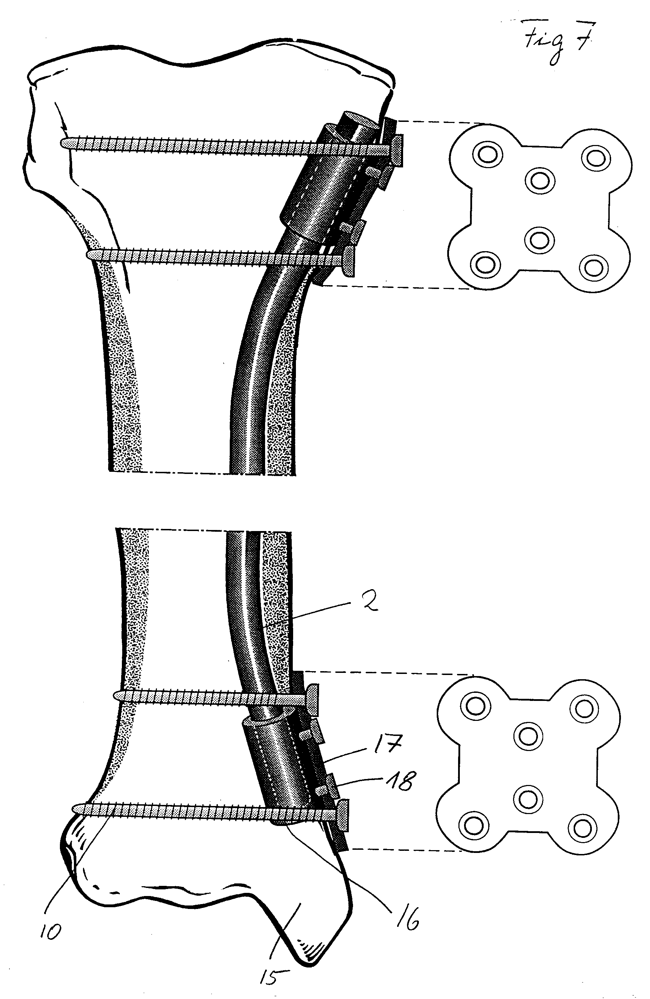

Die Ausführungsform nach Figur 7 zeichnet sich dadurch aus, daß sie eine besonders sichere endseitige Fixierung des Kraftträgers 2 gestattet, indem ein Aufnahmeteil 16 in Köcherform vorgesehen ist, das mittels einer Fixierplatte über Schrauben 10 am jeweiligen Knochen 15 befestigbar ist und vorzugsweise eine formschlüssige Aufnahme des Kraftträgerendes sicherstellt. Um eine eindeutige Ausschaltung von Rotations-, Kippungs- und Verkürzungseffekten zu gewährleisten, wird der Kraftträger 2 mittels einer Klemmschraube 18 im Aufnahmeteil 16 festgelegt, wobei diese Klemmschraube 18 durch die Fixierungsplatte 17 geführt und damit gut zugänglich ist. Anstelle eines köcherförmigen Gebildes kann als Aufnahmeteil 16 auch ein Ringelement vorgesehen sein, so daß der Kraftträger 2 das Aufnahmeteil 16 auch etwas durchgreifen kann.

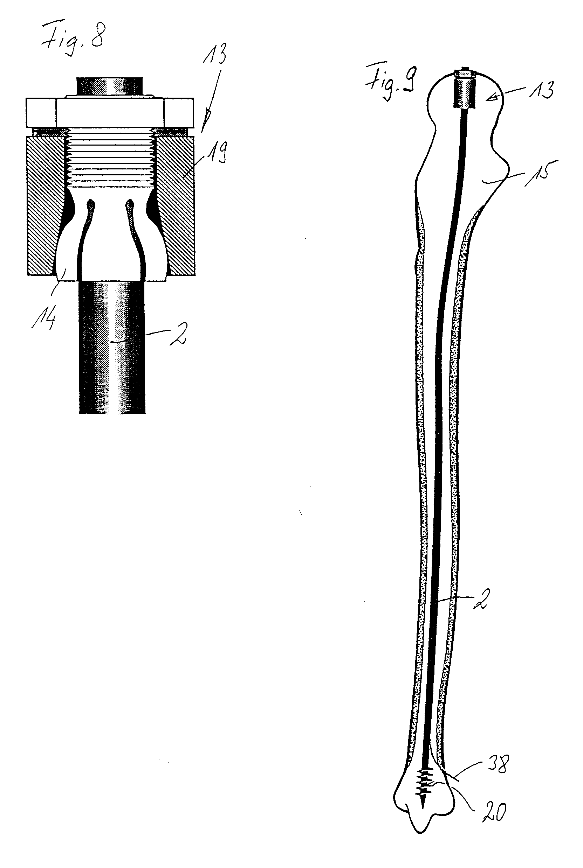

Figur 8 zeigt eine weitere Ausgestaltung einer Fixiereinheit nach der Erfindung in Form eines Klemmkonus 13. Dabei kann das lamellierte Innenteil 14 mittels des Schraubkonusteils 19 derart verengt werden, daß der Kraftträger 2 kraft- und formschlüssig umschlossen und fixiert wird. Der Vorteil einer Fixiereinheit in Form eines Klemmkonus besteht vor allem darin, daß ein derartiger Klemmkonus die Möglichkeit schafft, durch entsprechend längere Ausgestaltung eine Schienung des Kraftträgers und damit auch eine mechanische Verfestigung bis in den mittleren Schaftbereich hinein zu gewährleisten.

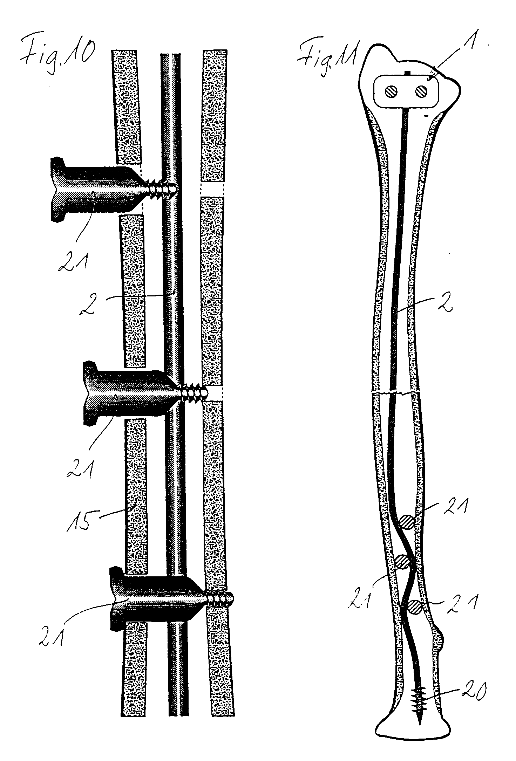

Figur 9 zeigt die Kombination einer Fixiereinheit in Form eines Klemmkonus 13 mit etwas verlängertem Schaftbereich mit einer Befestigung über ein Gewindeende 20 des Kraftträgers 2, das im spongiösen Knochen Halt findet. Mit dieser Anordnung ist problemfrei eine Sicherung gegen Verkürzung, Kippung und Seitverschiebung zu erzielen. Besonders vorteilhaft ist es, den Kraftträger 2 hohl auszubilden und den Innenraum des Kraftträgers zur Führung eines Spickdrahtes 38 zu verwenden. Dieser Draht 38 erbringt durch entsprechende Verankerung im Knochen 15 eine besonders hohe Positionierungssicherheit, insbesondere gegenüber Rotation. Die Austrittsstelle des Drahts 38 liegt dabei kurz vor Beginn des Gewindes 20.

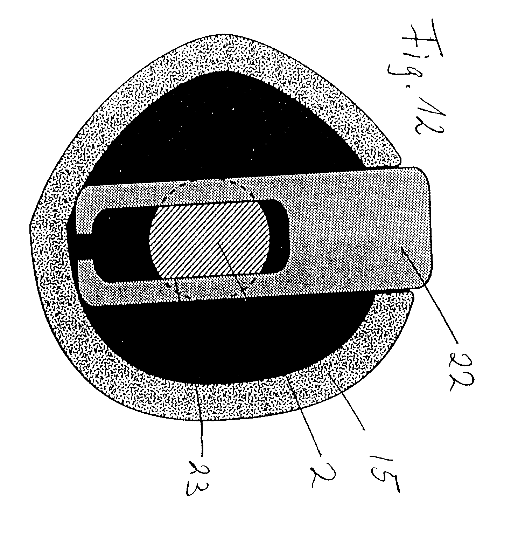

Figur 10 zeigt eine Variante einer Fixiereinheit, die insbesondere für relativ dünne Kraftträger, die beispielsweise einen Durchmesser von 2 mm bis 3 mm haben, geeignet ist. Diese Fixiereinheit besteht aus drei gegenseitig beabstandeten Schrauborganen 21, die bezüglich der Mittelachse des Kraftträgers 2 wechselseitig versetzt und unter Verformung des Kraftträgers 2 im Knochen 15 fixierbar sind.

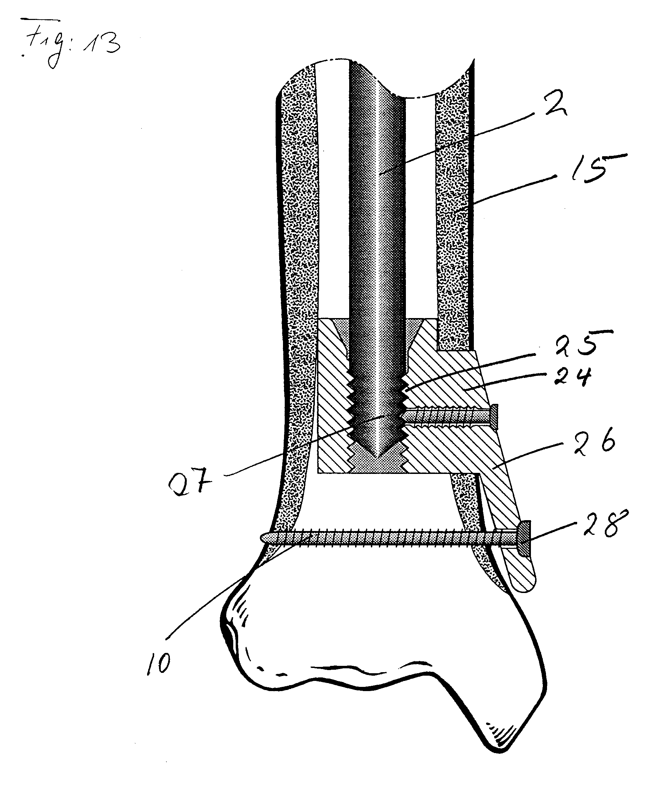

Die Darstellung nach Figur 11 zeigt, daß durch diese Verformung des Kraftträgers 2 über die Schrauborgane 21 eine einwandfreie, sich über einen größeren Bereich erstreckende Fixierung des Kraftträgers 2 möglich ist, wobei diese Fixiereinheit mit einer beliebigen der weiteren beschriebenen Fixiereinheiten kombinierbar ist, die im Bereich eines Endes des Kraftträgers 2 positioniert werden kann.

Figur 12 zeigt eine Ausführungsform einer Fixiereinheit, bei der der Kraftträger 2 seitliche Einkerbungen besitzt und mit einem Cliporgan 22 bezüglich des Knochens fixiert werden kann, das sich durch eine entsprechende Bohrung im Knochen erstreckt und bezüglich des Knochens 15 festlegbar ist. Da das jeweilige Cliporgan 22 formschlüssig in die entsprechenden Querkerbungen 23 des Kraftträgers 2 eingreift und das Cliporgan 22 mittels eines entsprechenden Klammergeräts über eine passende Knochenbohrung eingesetzt werden kann, ergibt sich die geforderte Sicherheit gegen Verkürzung, Kippung und Rotation ohne besondere Schwierigkeit. Es können auch mit gegenseitigem Abstand mehrere derartige Cliporgane vorgesehen sein.

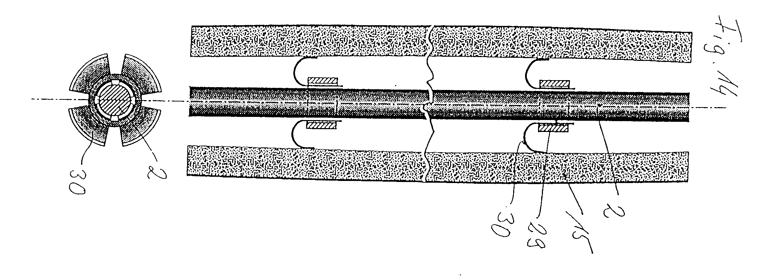

Figur 13 zeigt eine Fixiereinheit, die aus einem Ringorgan 24 mit Innengewinde 25 besteht, das in die Markhöhle des Knochens 15 eingebracht und bezüglich des Knochens über ein mit dem Knochen über Schrauben 10 verschraubtes Halteelement 26 fixiert ist. Dieses mit Innengewinde 25 versehene Ringorgan wirkt mit dem mit einem entsprechenden Außengewinde 27 ausgestatteten Ende des Kraftträgers 2 zusammen, der in das Ringelement 24 geschraubt werden kann. Um eine Rotationssicherung zu erzielen, kann entweder eine gegen Klemmung gehende Verschraubung vorgesehen sein, oder es wird eine Fixierung mittels einer Blockierschraube 28 vorgenommen, die sich durch das Ringteil 24 erstreckt und das Gewinde blockierend beaufschlagt.

Da bei der ungebohrten intramedullären Schienung der jeweilige Kraftträger relativ dünn ist bzw. einen vergleichsweise geringen Querschnitt besitzt, sind gemäß einem weiteren Aspekt der Erfindung zur Sicherstellung der mechanischen Anforderungen hinsichtlich Belastbarkeit und Frakturruhigstellung intramedulläre Maßnahmen zur Fixierung des Kraftträgers 2 relativ zur Knochenwandung vorgesehen, und diese Maßnahmen bestehen bevorzugt in der Bereitstellung eines Spreizmechanismus in Form mehrerer, auf den Kraftträger 2 aufgebrachter und bezüglich des Kraftträgers lagefixierter Spreizorgane.

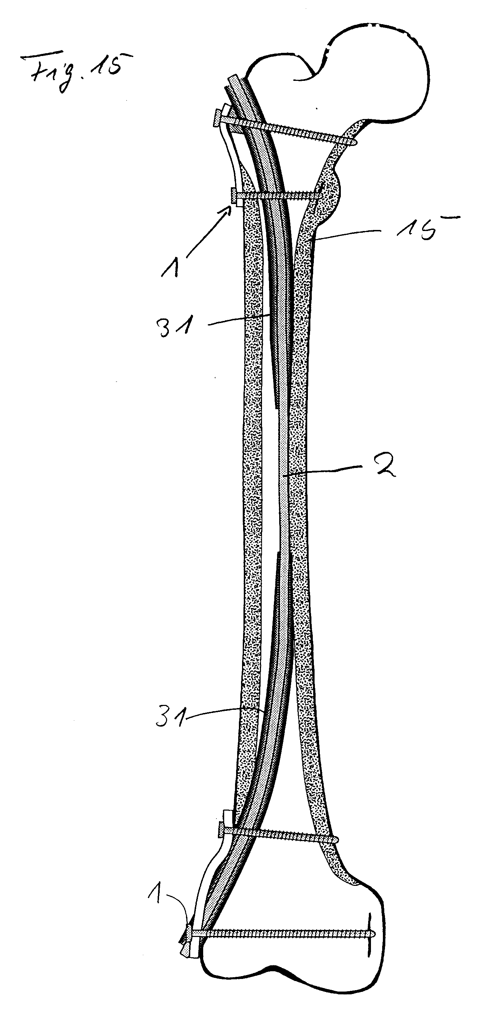

Figur 14 zeigt ein Beispiel derartiger Spreizorgane 29, deren Radialelemente elastisch ausgebildet sind und die erforderliche Zentrierung bzw. Stützung des Kraftträgers 2 relativ zum Knochen 15 erbringen. Die Querschnittsdarstellung zeigt die flächige Ausgestaltung der einzelnen Radialelemente, welche aufgrund dieser Gestalt die geforderte zentrierte und sichere Abstützung gewährleisten.

Möglich ist es auch, auf den jeweiligen Kraftträger 2 ein resorbierbares Implantat aufzubringen, wobei in diesem Falle die Fixation nur temporär wirksam ist, was aber in vielen Fällen durchaus erwünscht sein kann. Wichtig ist jedoch in diesem Falle die Lagefixierung in Längsrichtung des Kraftträgers 2.

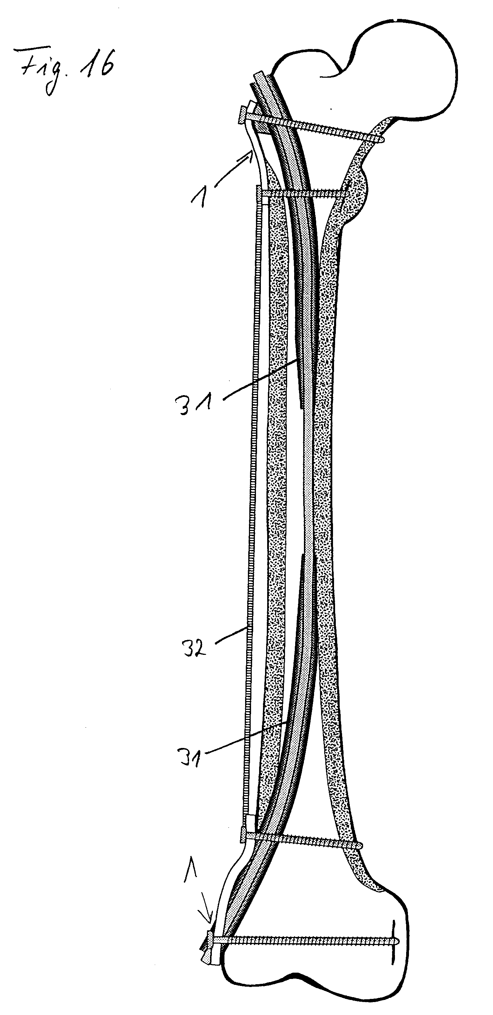

Figur 15 zeigt ein insbesondere auch für entsprechend dünne Kraftträger 2 geeignetes System, bei dem die Fixiereinheiten 1 jeweils mit einem Führungsrohr 31 ausgestattet sind, durch das sich der Kraftträger 2 erstreckt und in dem der Kraftträger 2 durch Klemmung eindeutig fixierbar ist.

Die Ausführungsform nach Figur 16 verdeutlicht, daß die Fixiereinheiten 1 gemäß der Erfindung auch mit außenseitigen Zuggurtungsplatten 32 zusammenwirken können, wobei sich diese Zuggurtungsplatte 32 zwischen zwei Fixiereinheiten 1 erstrecken oder an einer Fixiereinheit angebracht und am distalen Ende verschraubt sein kann. Auf diese Weise können Kombinations- bzw. Modellarsysteme geschaffen werden.

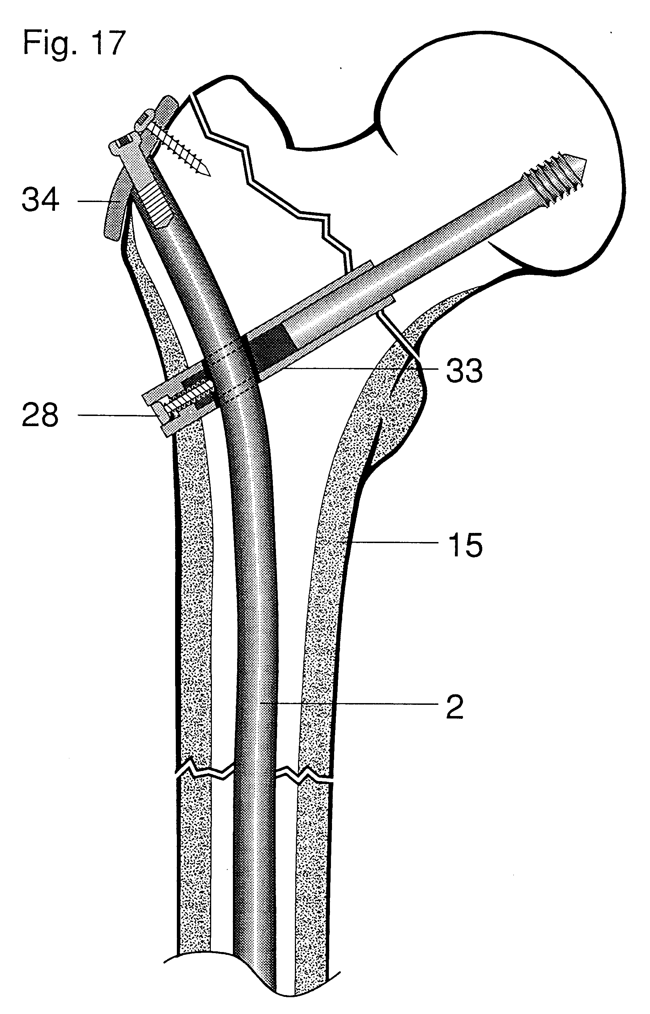

Figur 17 zeigt eine Variante der Erfindung, bei der der Kraftträger 2 einerseits in seinem Endbereich mittels einer Fixiereinheit nach der Erfindung festgelegt ist, andererseits eine Halte- oder Kopplungsstelle für ein weiteres Spann-Fixierelement 33 bildet, um beispielsweise auf diese Weise ein Zusammenwirken mit einem Blockierschraubensystem zu ermöglichen.

Der Kraftträger 2 erstreckt sich dabei durch eine entsprechende Bohrung eines Spannsystems, das durch Verschraubung im Knochen fixierbar ist, so daß die geforderten Druckkräfte auf die Fraktur aufgebracht werden können. Die Relativfixierung zwischen Kraftträger 2 und Fixierelement 33 erfolgt vorzugsweise mittels einer Blockierschraube 28.

Obwohl das Ende des Kraftträgers 2 mittels einer der vorstehend beschriebenen Fixiereinheiten positioniert und festgelegt werden könnte, ist bei dieser Ausführungsform nach Figur 17 das Ende des Kraftträgers 2 mit einer Befestigungsplatte 24 verschraubt, die wiederum am Knochen 15 befestigt ist.

Die Ausführungsbeispiele nach den Figuren 16 und 17 zeigen, daß für spezielle Frakturformen, die sich vom Schaftbereich bis in den Gelenkbereich ausdehnen, Systeme zur Verfügung gestellt werden können, die eine Kombination von Ruhigstellung der Schaftfraktur mit denen der gelenknahen Fraktur erlauben, wozu Hüftschrauben-Prinzipien mit dem Marknagelsystem kombiniert werden.

Bei herkömmlichen intramedullären Systemen ist von Nachteil, daß erforderliche Führungsinstrumente in Vor- und Rückwärtsbewegungen eingebracht und wieder entfernt werden mußten. Derartige Führungsinstrumente sind für die ungebohrten Systeme, das heißt für die Systeme mit vergleichsweise dünnem Kraftträger nicht vorgesehen. Im Zusammenhang mit den erfindungsgemäßen Fixierungssystemen ist vorgesehen, den jeweiligen Marknagel bereits so vorzubiegen, daß er über entsprechende seitliche Bohrungen eingeführt und am distalen Ende auch wieder herausgeführt und dann mittels Fixiereinheiten gemäß der Erfindung festgelegt werden kann. Möglich ist es auch, einen Führungsdraht zu verwenden, der mit dem jeweiligen Nagel kraftschlüssig kuppelbar ist, um ein problemfreies Nachziehen des Nagels zu ermöglichen.

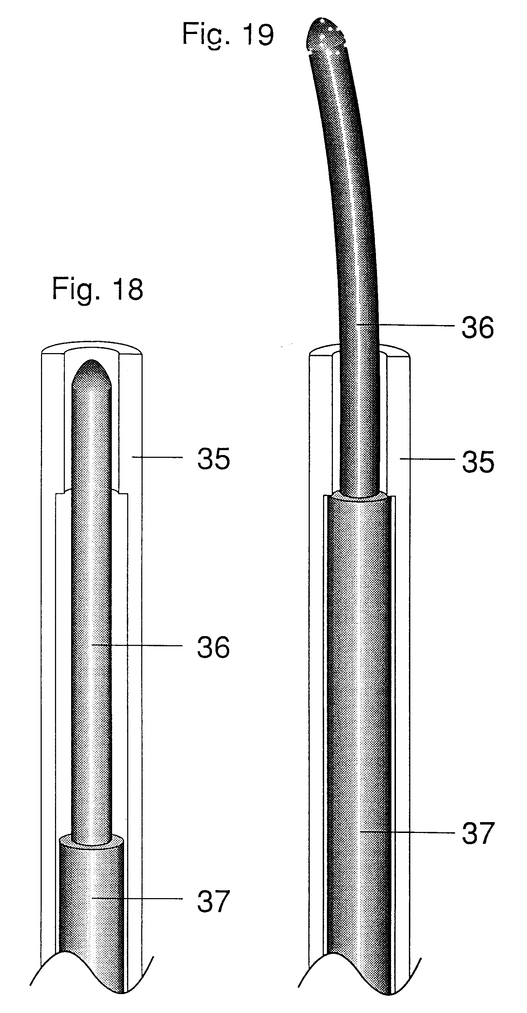

Die Figuren 18 und 19 zeigen ein Kraftträgersystem, das es ermöglicht, auch bei schwierigen intramedullären Versorgungen stark verschobene Fragmente aufzufädeln, was in manchen Fällen, beispielsweise im Oberschenkelbereich, bisher häufig erhebliche Schwierigkeiten verursacht.

Wie Figur 18 zeigt, ist der Marknagel in Form eines Rohres 35 ausgebildet, in dem ein Stab 37 verschiebbar angeordnet ist. Das Ende des Stabes 37 trägt einen Dorn 36, der - wie Figur 19 zeigt - aus dem Rohr 35 herausgeschoben und dann wieder zurückgezogen werden kann. Dieser Dorn 36 ist vorzugsweise im Bereich seiner Spitze gekrümmt, so daß er im ausgefahrenen Zustand ein verschobenes Fragment auffädeln kann. Die Krümmung des Dorns 36 wird aufgrund einer entsprechenden Vorspannung nur wirksam im ausgefahrenen Zustand, während der eingefahrene Dorn zumindest im wesentlichen im gestreckten Zustand vorliegt.

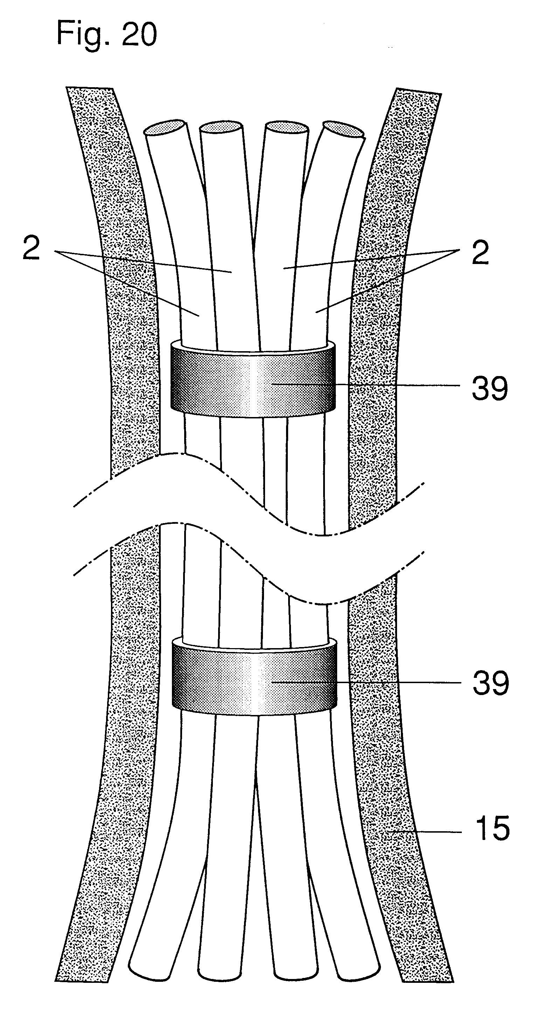

Die schematische Darstellung nach Figur 20 zeigt das Prinzip eines gebündelten Festigkeitsträgers, bestehend aus mehreren Einzel-Festigkeitsträgern 2, die durch Bandagenorgane 39 zu einer besonders stabilen Einheit zusammengefaßt sind. Die Querschnitte der einzelnen Festigkeitsträger 2 können dabei gleich aber in Abhängigkeit vom jeweiligen Anwendungsgebiet auch unterschiedlich gewählt werden. Der gegenseitige Abstand der Bündelungsbandagen 39 wird so gewählt, daß sich die gebündelten Kraftträger 2 in dem Bereich zwischen den Bandagen wie eine integrale Einheit verhalten, wobei aber bei dieser Ausführungsform der Vorteil besteht, daß die Einbringung der Kraftträger 2 erleichtert wird und daß durch Auswahl der entsprechenden Kraftträger und deren Anzahl die gewünschte Festigkeit vorgegeben werden kann. Die Anbringung der Bündelungselemente 39 kann mittels geeigneter Werkzeuge ohne besondere Probleme erfolgen.

### Bezugszeichenliste

- 1: Fixiereinheit
- 2: Kraftträger
- 3: Klemmbacke
- 4: Klemmbacke
- 5: Schraubspindel
- 6: Kugelgelenk
- 7: Klemmschale
- 8: Grundplatte
- 9: Druckplatte
- 10: Plattenschraube
- 11: Klemmschraube
- 12: Ausnehmung
- 13: Klemmkonus
- 14: Lamelliertes Innenteil
- 15: Knochen
- 16: Aufnahmeteil
- 17: Fixierplatte
- 18: Klemmschraube
- 19: Schraubkonusteil
- 20: Gewindeende
- 21: Schrauborgan
- 22: Cliporgan
- 23: Querkerbung
- 24: Ringorgan
- 25: Innengewinde
- 26: Halteelement
- 27: Außengewinde
- 28: Blockierschraube
- 29: Spreizorgan
- 30: Radialelement
- 31: Führungsrohr
- 32: Zuggurtungsplatte
- 33: Fixierelement
- 34: Befestigungsplatte
- 35: Rohr
- 36: Dorn
- 37: Stab
- 38: Spickdraht
- 39: Bündelungsmanschette

## Patentansprüche

1. Fixierungs- und Positionierungssystem für intramedulläre Kraftträger, insbesondere für vorbohrungsfrei in das jeweilige Knochenrohr einbringbare Marknägel, mit zumindest zwei gegenseitig beabstandeten Fixiereinheiten,
dadurch **gekennzeichnet,**
daß jede Fixiereinheit (1) als bezüglich des Knochens festlegbare, dem jeweiligen Kraftträger (2) durchdringungsfrei halternde, Rotations-, Kippungs- und Verkürzungseffekte ausschließende Einheit ausgebildet ist.

2. System nach Anspruch 1,
dadurch **gekennzeichnet,**
daß zumindest eine der Fixiereinheiten aus zwei Klemmbacken (3, 4) besteht, die über eine Schraubspindel (5) mit gegenläufigem Gewinde form- und kraftschlüssig mit dem Kraftträger (2) verspannbar sind, wobei insbesondere zumindest eine der Klemmbacken (3, 4) eine beweglich, insbesondere über ein Kugelgelenk (6) gelagerte Klemmschale (7) zur formschlüssigen Aufnahme des Kraftträgers (2) aufweist.

3. System nach Anspruch 1,
dadurch **gekennzeichnet,**
daß zumindest eine der Fixiereinheiten aus zwei gegeneinander verspannbaren, zwischen sich den jeweiligen Kraftträger (2) festlegenden Platten (8, 9) besteht, wobei eine der Platten als mit dem jeweiligen Knochen verschraubbare Grundplatte (8) und die andere Platte als gegen diese Grundplatte (8) über zumindest eine Klemmschraube (11) verspannbare Druckplatte (9) ausgebildet ist und vorzugsweise zumindest die Grundplatte (8) oder die Druckplatte (9) eine Ausnehmung (12) zur form- und kraftschlüssigen Teilaufnahme des Kraftträgers (2) aufweist.

4. System nach Anspruch 1,
dadurch **gekennzeichnet,**
daß zumindest eine der Fixiereinheiten aus einem ring- oder köcherförmigen Aufnahmeteil (16) besteht, das mit einer Fixierungsplatte (17) zur Befestigung am Knochen (15) verbunden ist und zumindest eine sich in den Ring- oder Köcherraum erstreckende, am Kraftträger (2) angreifende Klemmschraube (18) aufweist.

5. System nach Anspruch 1,
dadurch **gekennzeichnet,**
daß zumindest eine der Fixiereinheiten aus einem bezüglich des Knochens fixierbaren und vom Kraftträger (2) durchsetzbaren Klemmkonus (13) besteht, dessen lamelliertes Innenteil (14) über ein Schraubkonusteil (19) verengbar und gegen den Kraftträger (2) verspannbar ist und / oder eine der Fixiereinheiten von dem Endteil des jeweiligen Kraftträgers (2) gebildet ist, das die Form eines relativ großen, im spongiösen Knochenbereich verankerbaren Gewindes (20) besitzt.

6. System nach Anspruch 1, insbesondere für relativ dünne, elastisch verformbare Kraftträger,
dadurch **gekennzeichnet,**
daß zumindest eine der Fixiereinheiten wenigstens drei gegenseitig beabstandete Schrauborgane (21) umfaßt, die bezüglich der Mittelachse des Kraftträgers (2) wechselseitig versetzt und unter Verformung des Kraftträgers (2) im Knochen (15) fixierbar sind, wobei die Schrauborgane (21) zwischen ihren beiden im Knochen (15) fixierbaren Enden einen auf den Kraftträger (2) keilartig wirkenden Bereich aufweisen.

7. System nach Anspruch 1,
dadurch **gekennzeichnet,**
daß zumindest eine der Fixiereinheiten aus einem Cliporgan (22) besteht, das in eine Querkerbung (23) des Kraftträgers (2) formschlüssig einbringbar und in einer Knochenbohrung fixierbar ist.

8. System nach Anspruch 1,
dadurch **gekennzeichnet,**
daß zumindest eine der Fixiereinheiten aus einem ein Innengewinde (25) aufweisenden, in die Markhöhle einbringbaren Ringorgan (24) besteht, das über ein Halteelement (26) am Knochen (15) fixierbar ist und eine Schraubaufnahme für das mit einem entsprechenden Außengewinde (27) versehene Ende des Kraftträgers (2) bildet, wobei in das Ringorgan (24) vorzugsweise quer zur Gewindelängsachse eine Blockierschraube (28) einschraubbar ist.

9. System, insbesondere nach einem der vorhergehenden Ansprüche,
dadurch **gekennzeichnet,**
daß auf den Kraftträger (2) verschiebegesichert aufbringbare, insbesondere aufschiebbare Spreizorgane (29) vorgesehen sind, deren sich an der Knocheninnenwand abstützende Radialelemente (30) elastisch ausgebildet sind, wobei die Spreizorgane insbesondere aus einem resorbierbaren Implantat bestehen.

10. System nach einem der vorhergehenden Ansprüche,
dadurch **gekennzeichnet,**
daß mit zumindest einer Fixiereinheit ein Führungsrohr (31) für den Kraftträger (2) verbunden ist, das sich vorzugsweise über einen längeren Abschnitt des Kraftträgers (2) erstreckt und in dem der Kraftträger (2) durch Klemmung fixierbar ist.

11. System nach einem der vorhergehenden Ansprüche,
dadurch **gekennzeichnet,**
daß zwei beabstandete, insbesondere im Bereich der Enden des Kraftträgers (2) angeordnete Fixiereinheiten (1) durch eine außenliegende Zuggurtungsplatte (32) miteinander verbunden sind.

12. System nach einem der vorhergehenden Ansprüche,
dadurch **gekennzeichnet,**
daß zumindest eine Fixiereinheit (1) relativ zum Kraftträger (2) unter Druckausübung auf die jeweilige Fraktur insbesondere über eine von außen betätigbare Schraubverbindung verstellbar ist.

13. System nach einem der vorhergehenden Ansprüche,
dadurch **gekennzeichnet,**
daß die Fixiereinheit aus einer knochenaußenseitig mit dem Kraftträgerende verbindbaren Befestigungsplatte (34) und einem dazu beabstandeten, im wesentlichen quer zum Kraftträger (2) verlaufenden und von diesem durchsetzten Fixierelement (33) besteht, das im Knochen, insbesondere im Hüftgelenksknochen durch Verschraubung fixierbar ist, wobei der Kraftträger (2) eine Gegenverankerung für das Fixierelement (33) bildet und der Kraftträger vorzugsweise aus einem Rohr (35) und einem darin verschiebbaren Stab (37) besteht, an dessen Ende ein aus dem Rohr (35) durch Längsverschiebung des Stabes (37) ausfahrbarer Dorn (36) vorgesehen ist, dessen freier Endbereich rückstellbar gekrümmt ausgebildet ist oder der Hohlraum des Rohres zur Führung eines Spickdrahtes dient, dessen am distalen Ende austretendes Ende zur Rotationsverhinderung im Knochen verankerbar ist, wobei die Austrittstelle des Spickdrahtes vor der Fixiereinheit gelegen ist, die von einem relativ großen, im spongiösen Knochenbereich verankerbaren Gewinde (20) gebildet ist.

14. System nach einem oder mehreren der vorhergehenden Ansprüche,
dadurch **gekennzeichnet,**
daß in den Knocheninnenraum zwei oder mehr nebeneinander angeordnete Kraftträger (2) von vergleichsweise geringem Durchmesser angeordnet sind und daß diese Mehrzahl von Kraftträgern (2) durch Umschließungsbandagen (39) zu einer eine hohe Festigkeit besitzenden Bündelanordnung zusammengefaßt sind, wobei die Durchmesser der zu einem Bündel zusammengefaßten Kraftträger (2) vorzugsweise unterschiedlich sind.
